# EUROPEAN PATENT SPECIFICATION

(11) **EP 3 752 593 B1**
(45) Date of publication and mention of the grant of the patent: **01.12.2021**
(21) Application number: 19707945.2
(22) Date of filing: 08.02.2019
(51) Int. Cl.: C12M 1/12, C12M 1/24, C12M 1/34

(54) **REMOTE MONITORING SYSTEM FOR CELL CULTURE**
FERNÜBERWACHUNGSSYSTEM FÜR ZELLKULTUREN
SYSTÈME DE SURVEILLANCE À DISTANCE POUR LA CULTURE CELLULAIRE

(30) Priority: 12.02.2018 US 201862629483 P
(43) Date of publication of application: 23.12.2020
(73) Proprietor: Corning Incorporated, Corning, New York 14831 (US)
(72) Inventor: MARTIN, Gregory Roger, Acton, Maine 04001 (US); WALL, Joseph Christopher, Southborough, MA 01772 (US); WRIGHT, Alan Craig, Greenville, New Hampshire 03048 (US)
(74) Representative: Elkington and Fife LLP
(86) International application number: PCT/US2019/017185
(87) International publication number: WO 2019/157263

(56) References cited:
- EP-A1- 3 144 379
- EP-B1- 0 667 894
- WO-A1-2008/108092
- US-A1- 2008 113 404

## Description

### FIELD

The present disclosure relates generally to cell culture monitoring, and more specifically to a non-invasive monitoring system designed to take measurements in an adherent cell vessel.

### BACKGROUND

Cell cultures are widely used to provide an artificial environment for cell growth. In some cases, a stacked cell culture vessel may provide an increased area for cell growth over single layer dishes. Cells may grow in suspension or attached to a cell culture vessel surface. The processing of cell cultures includes two principal activities, monitoring cell growth and health (confluence and morphology) and ensuring a suitable environment for cell growth (e.g., pH, glucose, and lactate levels). Production cost of cell cultures is extremely high due to low yield, high labor cost, intensive manual work flow, and costly clean room environments where processing is often performed. Monitoring methods of cell cultures are a significant factor for increasing yield and decreasing costs.

Current methods for both viewing cells and measuring analytes can be time consuming and require direct access to the vessel, which risks breaking the sterility of the vessel's environment. Scientists often utilize the naked eye or microscopes to view the confluence of cells. Unfortunately, these methods require direct access to the vessel, which often slows or stops cell growth. Moreover, direct access methods make it difficult or impossible to automate the process. For example, when a stacked cell culture vessel is used, only the exterior layers or near-exterior layers can be monitored, and the status of the interior layers must be estimated without direct measurements.

Cell culture processing is presently monitored (e.g., the presence of certain analytes) through invasive and semi-invasive methods which utilize components such as probe sensors or patches. These methods require some type of contact with the invasive or semi-invasive components within the cell growth environment even though it is desired to allow the systems to operate as a closed system. Monitoring methods are often insufficient and production environments rely on process development techniques for timing the feeding and harvest of cells, which still require manual monitoring. EP 3144379 A1 describes a culture observation apparatus comprising a plurality of stacked layers and an image-acquisition portion.

A closed system with non-invasive monitoring may allow for use of automation to better control cell culture media compositions and cell growth and health. Closed systems can maintain sterility throughout growth, which reduces the requirements and cost of a clean room. Further, real-time monitoring data may be transmitted to a user in a remote location, which can reduce the need of manual labor.

### SUMMARY

A remote monitoring system for non-invasive measurement of a cell culture is provided. The system includes a plurality of cell culture layers comprising a cell culture chamber configured to operate as a closed-system, the at least one cell culture chamber having at least one surface to which cells adhere. The system further includes at least one monitoring layer comprising an outer wall surrounding a monitoring layer cell culture chamber configured to operate as a closed-system and having at least one surface to which cells adhere, the at least one monitoring layer comprising at least one indentation in the outer wall. The system also includes at least one monitoring module disposed in at least one of the at least one indentation and comprising at least one of a confluence monitor and an analyte monitor.

Further scope of the applicability of the described methods and systems will become apparent from the following detailed description, claims, and drawings. The detailed description and specific examples are given by way of illustration only, since various changes and modifications within the spirit and scope of the description will become apparent to those skilled in the art.

### BRIEF DESCRIPTION OF THE DRAWINGS

A further understanding of the nature and advantages of the present disclosure may be realized by reference to the following drawings. In the appended figures, similar components or features may have the same reference label. Further, various components of the same type may be distinguished by following the reference label by a dash and a second label that distinguishes among the similar components. If only the first reference label is used in the specification, the description is applicable to any one of the similar components having the same first reference label irrespective of the second reference label.
FIG. 1 illustrates a perspective view of an example of a monitoring layer for non-invasive measurement of a cell culture that supports remote monitoring in accordance with embodiments of the present disclosure.
FIG. 2 illustrates an example of a monitoring module in accordance with embodiments of the present disclosure.
FIG. 3 illustrates a perspective view of an example of a stacked cell culture vessel system for non-invasive measurement of a cell culture that supports remote monitoring in accordance with embodiments of the present disclosure.
FIG. 4 illustrates an example of an indentation of a monitoring layer in accordance with embodiments of the present disclosure.
FIG. 5 shows a cross section of the monitoring module of FIG. 2 taken along the confluence monitor and illustrates an example of a confluence monitor in accordance with embodiments of the present disclosure.
FIG. 6 shows a cross section of the monitoring module of FIG. 2 taken along the analyte monitor and illustrates an example of an analyte monitor in accordance with embodiments of the present disclosure.
FIG. 7 shows a cross section of the monitoring module of FIG. 2 taken along the analyte monitor and illustrates an example of an analyte monitor in accordance with embodiments of the present disclosure.

### DETAILED DESCRIPTION

The singular forms "a," "an" and "the" include plural referents unless the context clearly dictates otherwise. The endpoints of all ranges reciting the same characteristic are independently combinable and inclusive of the recited endpoint. All references are incorporated herein by reference.

As used herein, "have," "having," "include," "including," "comprise," "comprising" or the like are used in their open ended sense, and generally mean "including, but not limited to."

All scientific and technical terms used herein have meanings commonly used in the art unless otherwise specified. The definitions provided herein are to facilitate understanding of certain terms used frequently herein and are not meant to limit the scope of the present disclosure.

The present disclosure is described below, at first generally, then in detail on the basis of several exemplary embodiments. The features shown in combination with one another in the individual exemplary embodiments do not all have to be realized. In particular, individual features may also be omitted or combined in some other way with other features shown of the same exemplary embodiment or else of other exemplary embodiments.

In practice, cell culture systems that allow for certain measurements to be completed in real time without disturbing the cells, or in other words, a closed system, may facilitate maintaining a sterile cell growth environment. For example, a monitoring system that is external to a cell culture chamber may provide a non-invasive method for measuring cell status, such as cell growth and health, without directly contacting the cells and without contaminating the growth environment. As used herein, the term "closed system" refers to a system wherein the contents of the system are not open to the surrounding atmosphere. The system may include a closure apparatus, such as a cap, which limits or prevents the introduction of contaminants from the surrounding atmosphere. The system may be, but is not necessarily, sealed to ensure sterility of the contents of the system.

As described herein, a cell culture vessel may include a monitoring layer including at least one indentation, the at least one indentation being configured to receive at least one of optical technology (e.g., micro lens arrays and waveguides) and spectral analytical technology. Embodiments of the present disclosure further include a monitoring module including at least one of optical technology and spectral analytical technology. As will be described in more detail below, the monitoring module may include optical technology and spectral analytical technology integrated into the monitoring module. The cell culture vessels described herein may be adherent cell culture vessels generally including a planar surface on which cells adhere while being cultivated. Embodiments of the present disclosure enable the monitoring of cell confluence and measuring analytes with spectral interrogation that illuminates, receives, and processes signature wavelengths. A communication component may be utilized to transmit monitoring data from the monitor or monitoring module to a user in a remote location. This configuration may be implemented in single use or multi-use stacked vessels.

Embodiments of the present disclosure provide for closed-system operation of an adherent cell culture vessel with a monitoring module disposed external to cell culture layers. Embodiments of the present disclosure allow for the transmission of cell status from the monitoring layer to a user in a remote location. The monitoring layer may be positioned between other cell culture layers within a stacked cell culture vessel, and take measurements of cell culture chambers of the various layers of the stack. The closed system remains sterile and able to continuously grow cells, for example by remaining in an incubator, while taking real-time cell status data.

Having a monitoring module positioned external to a cell culture chamber may maintain sterility and enable remote and automated control of the process. By remotely monitoring cell confluence, embodiments of the present disclosure enable an operator to increase the yield in cell processing by optimizing the timing of next steps in cell production, thus reducing handling and reducing operating costs. This disclosure provides a mechanism to automate system controls allowing the operator to be a less skilled technician, lowering labor costs. With the external and remote implementation, the disclosure provides the main component to a closed cell production system that may be operated in a less costly environment.

The monitoring layer as described herein may be made of polystyrene. In conjunction with the monitoring module, the monitoring layer enables two monitoring functions of the cell growth areas: cell confluence and analyte measurement. The confluence monitor may employ a dual lens system with a mirror formed within the monitoring module, and an attached camera may provide light, image capture, magnification, and image delivery to a user. The analyte monitor may include a spectral analytical technology system and may further include a waveguide system with diffraction grating and lens in the monitoring module. Fibers for excitation and emission may be attached to the monitoring module and may also be connected to a spectral sensor system.

An exemplary confluence monitor may employ a dual lens system with a mirror for reflecting light to a cell growth surface with a cell cultuer chamber for illumination and image capture. The camera may provide the light and image capture function. Light waves or beams may travel through the lens to the mirror where it is focused on an area within the cell growth area. The illuminated image is then received by the camera once passing through the lens.

An exemplary analyte monitor may include a waveguide array. The monitor may employ dual optical ports where one port may be for excitation light and the other port may be for emission light. The excitation light may travel along the light guide (e.g., waveguide) to the diffraction grating and lens where it reflects off the diffraction grating into the media of a cell culture chamber. The emission fiber may receive the light from the excitation state of the media and deliver the excitation light to the spectral sensor (e.g., detector) to produce an emission or adsorption spectrum. The spectral sensor may include a 2D detector array system.

Embodiments of the present disclosure are initially described in the context of a cell culture system. Embodiments of the present disclosure are further illustrated by and described with reference to apparatus diagrams and system diagrams that relate to non-invasive remote measurements.

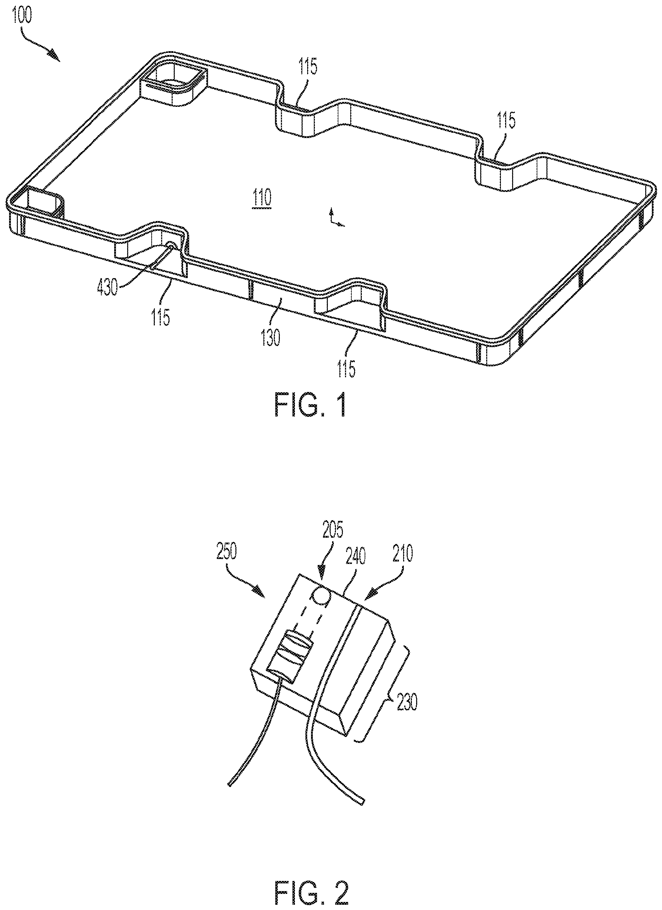

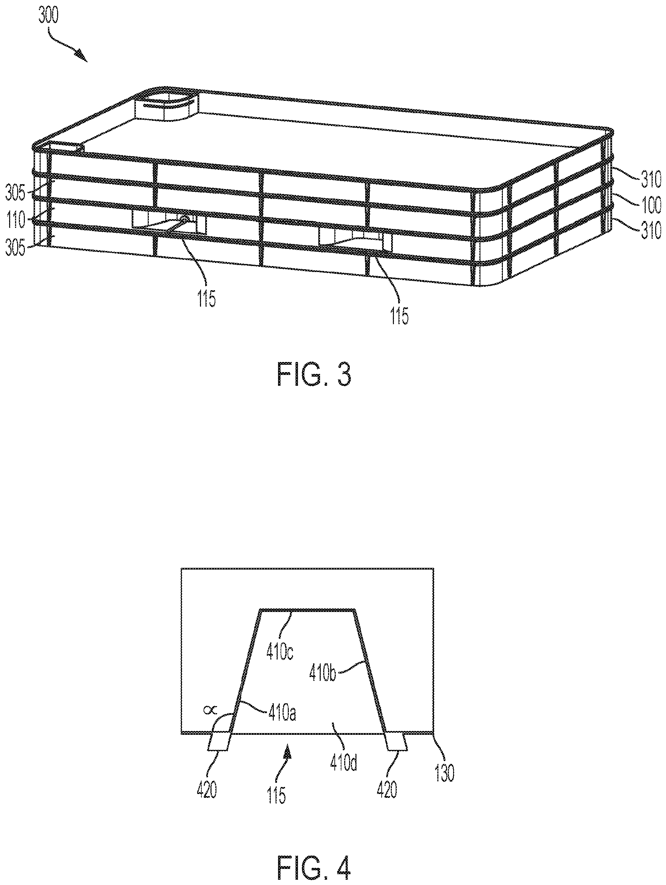

FIG. 1 shows a perspective view of a monitoring layer for non-invasive measurement of cell culture chambers that supports remote monitoring in accordance with embodiments of the present disclosure. The monitoring layer 100 includes an outer wall 130 surrounding a cell culture chamber 110, and at least one indentation 115 extending inward from the outer wall 130 toward the interior of the cell culture chamber 110. While the monitoring layer 100 shown in FIG. 1 includes four indentations 115, it should be appreciated that monitoring layers 100 in accordance with embodiments of the present disclosure may include any number of indentations 115. As will be explained in more detail below, the indentations 115 are configured to receive a monitoring module 250. As such, the indentations 115 and the monitoring module 250 may have corresponding shapes. As will further be explained in more detail below, the monitoring layer 100 may also include retaining features which cooperate with the monitoring module 250 to maintain the monitoring module 250 in the indentations 115. The monitoring layer 100 may be configured to operate in a wide temperature range, for example the monitoring layer 100 may operate in an incubator configured for cell growth. In some examples, the monitoring layer 100 may be part of a stacked cell culture vessel as shown in FIG. 3.

FIG. 2 illustrates a monitoring module in accordance with embodiments of the present disclosure. As described herein, the monitoring module 250 may include a head portion 230 having a front face 240 which is configured to contact an inner wall 410c of an indentation 115 of the monitoring layer 100. The monitoring module 250 further includes at least one of a confluence monitor 205 and an analyte monitor 210. It should be appreciated that the monitoring module 250 may include one of a confluence monitor 205 and an analyte monitor 210 or, alternatively, as shown in FIG. 2, may include both of a confluence monitor 205 and an analyte monitor 210. The confluence monitor 205 may be configured to measure cell status in the cell culture chamber 110 of the monitoring layer 100, or may be configured to measure cell status in a cell culture chamber 305 of a cell culture layer 310 positioned above or below the monitoring layer 100. Similarly, the analyte monitor 210 may be configured to monitor analytes in the cell culture chamber 110 of the monitoring layer 100, or may be configured to monitor analytes in a cell culture chamber 305 of a cell culture layer 310 positioned above or below the monitoring layer 100. Where the monitoring module 250 includes both a confluence monitor 205 and an analyte monitor 210, both monitors 205, 210 may be configured to monitor the cell culture chamber 110 of the monitoring layer 100, or both monitors 205, 210 may be configured to monitor at least one cell culture chamber 305 of a cell culture layer 310 positioned above or below the monitoring layer 100. Optionally, where the monitoring module 250 includes both a confluence monitor 205 and an analyte monitor 210, one of the confluence monitor 205 and the analyte monitor 210 may be configured to monitor the cell culture chamber 110 of the monitoring layer 100 and the other of the confluence monitor 205 and the analyte monitor 210 may be configured to monitor at least one cell culture chamber 305 of a cell culture layer 310 positioned above or below the monitoring layer 100.

According to embodiments of the present disclosure, the monitoring module 250 may have a shape corresponding to the shape of the indentation 115 of the monitoring layer 100 such that the monitoring module 250 may be received into the indentation 115. As shown in FIG. 4, the indentation 115 may have sidewalls 410a and 410b and an inner wall 410c. The sidewalls 410a, 410b may extend from the outer wall 130 of the monitoring layer 100 to the inner wall 410c of the indentation 115 at an angle α that is greater than about 90 degrees such that the indentation 115 has an isosceles trapezoid shape. The head portion 230 of the monitoring module 250 may have a corresponding isosceles trapezoid shape, or may have an orthogonal shape where the front face 240 of the head portion 230 has a width that is no greater than the width of the inner wall 410c of the indentation 115. Alternatively, the sidewalls 410a, 410b may extend perpendicular to the outer wall 130 of the monitoring layer 100 and parallel to each other. As such, the head portion 230 of the monitoring module 250, such as is illustrated in FIG. 2, may have an orthogonal shape which corresponds to the shape formed by the sidewalls 410a, 410b extending perpendicular to the outer wall 130. Alternatively, the sidewalls 410a, 410b may have a concave shape and the head portion 230 of the monitoring module 250 may have a rounded features configured to be received in the concave-shaped sidewalls 410a, 410b of the indentation 115. The shapes of the indentations 115 of the monitoring layer 100 and the head portion 230 of the monitoring module 250 discussed above are meant merely as examples. The indentations 115 may have any shape and the monitoring module 250 may have any corresponding shape such that the monitoring module 250 may be received in the indentation 115 and that the front face 240 of the head portion 230 contacts the inner wall 410c of the indentation 115.

The confluence monitor 205 may optically capture the cell status of the cells in the cell culture chamber 110, 305 and the analyte monitor 210 may optically capture analyte status in the cell culture chamber 110, 305. The confluence monitor 205 and the analyte monitor 210 may include a communication component for transmitting data, such as cell status data or analyte status data, from the monitors to a remote location via a wired communication network or a wireless communication network. For example, the communication component of each monitor may include a Wi-Fi transceiver.

FIG. 3 shows a perspective view of a stacked cell culture vessel system 300 that can be used, in conjunction with a monitoring module 250, for non-invasive measurement of a cell culture chamber 105, 305 in accordance with embodiments of the present disclosure. The stacked cell culture vessel system 300 may include a plurality of cell culture layers 310 and at least one monitoring layer100.

As illustrated, the stacked cell culture vessel system 300 may include any number of cell culture layers 310 and any number of monitoring layers 100. As shown in FIG. 3, the cell culture vessel system 300 may include a cell culture layer 310 below a monitoring layer 100 and a cell culture layer 310 above the monitoring layer 100. Where the cell culture vessel system 300 includes a plurality of monitoring layers 100, the system 300 may include any number of cell culture layers 310 between any two of the plurality of monitoring layers 100. For example, the cell culture vessel system 300 may include between 1 and 50 cell culture layers 310 between each monitoring layer 100, such as between or 2 and 40 cell culture layers 310, or between 3 and 35 cell culture layers 310, or between 5 and 30 cell culture layers 310, or even between 10 and 25 cell culture layers 310 between each monitoring layer 100, and all values therebetween. It should be appreciated that the number of cell culture layers 310 between different sets of the plurality of monitoring layers 100 may vary within the same stacked cell culture vessel system 300. Additionally, the stacked cell culture vessel system 300 may be configured to operate over a wide temperature range such as in an incubator at a temperature designed for cell growth.

FIG. 4 further illustrates exemplary retaining features in accordance with embodiments of the present disclosure. As shown, the outer wall 130 of the monitoring layer 100 includes clips 420 at the edges of the opening formed by the sidewalls 410a and 410b of the indentation 115. The clips 420 are configured to fit into a corresponding receptor on the monitoring module 250, thus maintaining the monitoring module 250 within the indentation 115. Another exemplary retaining feature is shown in FIG. 1. As shown, a base portion 410d of the indentation 115 includes a raised channel 430. The raised channel 430 is configured to fit into a corresponding notch on the bottom of the monitoring module 250, thus maintaining the monitoring module 250 within the indentation 115. As another option, the retaining feature may be a biased retention clip (not shown) on at least one surface of the monitoring module 250. The biased retention clip may have a similar design and function as those known to be used for telephone line connectors and Ethernet cable connectors. The indentations may include at least one clip groove (not shown) which receives a corresponding biased retention clip on a surface of the monitoring module 250 and which, in conjunction with the biased retention clip, limits motion of the monitoring module 250 and maintains the monitoring module 250 within the indentation 115.

According to embodiments of the present disclosure, the confluence monitors 205 and the analyte monitors 215 may capture the cell status of the cells and analyte status of the media in cell culture chambers 110, 305, including inter-layer measurements and monitoring. In some cases, a single confluence monitor 205 may monitor cell status of the cells of multiple stacked cell culture chambers 110, 305, or a single analyte monitor 215 may monitor analyte status of the media of multiple stacked cell culture chambers 110, 305.

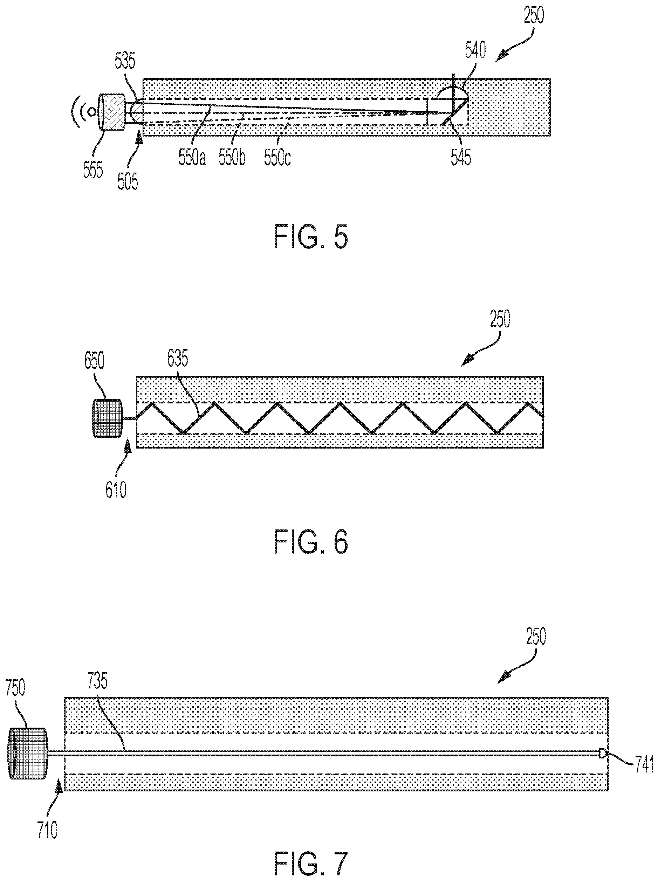

FIG. 5 shows a cross section of the monitoring module of FIG. 2 taken along the confluence monitor and illustrates an example of a confluence monitor in accordance with embodiments of the present disclosure. According to embodiments of the present disclosure, the confluence monitor 505 may take measurements of the cells in a cell culture chamber 110, 305 by any optical means. For example, the confluence monitor 505 may include a 2D imaging array to monitor cells in the cell culture chamber 110, 305. Alternatively, the confluence monitor 505 may include a multi-lens (e.g., dual lens) system with at least one mirror and at least one camera. FIG. 5 shows an exemplary confluence monitor 505 including light beams 550, first lens 535, second lens 540, and mirror 545 that may be configured to use a number of illumination options (e.g., reflected light illumination, epiillumination, dark field illumination, light field illumination, etc.) to observe the cells. Light beams 550 may be transmitted from a camera 555 through the first lens 535, where the light beams 550a-550c may be refracted and focused towards mirror 545. Once the light beams 550 contact the mirror 545, the light beams may be reflected at any angle, for example about 90 degrees, to be directed through the second lens 540 into a cell culture chamber 110, 305 to measure the confluence of cells. The camera 555 may capture the illuminated cells to produce an image of their real-time confluence that may be used to monitor cell growth over time. FIG. 5 shows an example of a confluence monitor 505 designed to image at least one cell culture chamber 305 above or below the monitoring layer 100. As media in the cell culture chambers 110, 305 may affect image quality, the confluence monitor 505 may take measurements of the cell culture chamber 305 above the monitoring layer 100 in order to image the cells on the side of the cell culture chamber 305 that has less media.

Optionally, the confluence monitor 505 may include a fiber probe (e.g., a dual clad fiber two multi-mode fibers (MMFs), or a multicore fiber) to direct light beams to the cell culture chamber 110, 305 and to transmit cell images to the camera 555. Additionally, image magnification to monitor cell confluence may be performed external to the monitoring module 250. For example, a light pipe may be used within the confluence monitor 505 to transfer the cell surface image without magnification to an external microscope at a location remote to the monitoring module 250.

FIG. 6 shows a cross section of the monitoring module of FIG. 2 taken along the analyte monitor and illustrates an example of an analyte monitor in accordance with embodiments of the present disclosure. According to embodiments of the present disclosure, the confluence monitor 610 may take measurements of the health of the cells by measuring the composition of the media within a cell culture chamber 110, 305 by any spectral means (e.g., Raman spectroscopy). The analyte monitor 610 may include a waveguide 635 (e.g., a light pipe) and detector 650. The waveguide 635 deliver light to the media within the cell culture chamber 110, 305. Optionally, the analyte monitor 610 may include a diffraction grating and lens which may receive excited light from the waveguide 635 and direct the excited light to the media within the cell culture chamber 110, 305. Excited light may be produced in a number of ways. Based on the composition of the media, distinct emission spectrums will be given off and captured by the detector 650. The detector 650 may transmit the captured emission or adsorption spectrum to a user. The user may use software to determine the composition of the media based on the emission or adsorption spectrums. Some examples of analytes that may be measured by analyte monitor 610 include glucose, lactose, and glutamine.

According to embodiments of the present disclosure, the analyte monitor 610 may include a light emitting diode (LED) or laser. The LED or laser may be paired with a photodiode detector within the analyte monitor 610.

FIG. 6 shows an example of an analyte monitor 610 designed to image the cell culture chamber 110 of the monitoring layer 100. However, as discussed above, the analyte monitor 610 may be designed to monitor at least one cell culture chamber 305 above or below the monitoring layer 100. A diffraction grating and lens may be utilized to direct light in the waveguide 635 to at least one cell culture chamber 305 above or below the monitoring layer 100. It is preferable for the analyte monitor 610 to transmit excited light into the media while passing through as few other materials as possible in order to produce a clean emission spectrum.

FIG. 7 shows a cross section of the monitoring module of FIG. 2 taken along the analyte monitor and illustrates an example of an analyte monitor in accordance with embodiments of the present disclosure. As shown, the analyte monitor 710 may include a fiber probe 735 (e.g., a dual clad fiber two multi-mode fibers (MMFs), or a multicore fiber), a lens 741, and a detector 750. The lens 741 may be integrated to the fiber end using a fiber lens making process. The fiber probe 735 may direct excited light through the lens 741 and to the media within the cell culture chamber 110, 305. Optionally, the analyte monitor 710 may include a mirror which may receive excited light from the fiber probe 735 and direct the excited light to the media within the cell culture chamber 110, 305. Alternatively, the fiber probe 735 may be bent about 90 degrees to direct a light beam through the lens 741 and to the media within the cell culture chamber 110, 305. When the fiber probe 735 is a dual clad fiber, the central inner core may be used to transmit a light beam to the media, and the outer core may be used to capture the Raman-scattered light from the media. The central inner core may be a single-mode or a multimode core. When the fiber probe 735 includes two MMFs, one MMF may transmit a light beam to the media, and the other MMF may capture the Raman-scattered light from the media. When the fiber probe 735 is configured with a multicore fiber, one core (e.g., the core in the center) may transmit a light beam to the media, and the other cores may capture the Raman-scattered light from the media.

Excited light may be produced in a number of ways. For example, the analyte monitor 710 as described above may include a light emitting diode (LED) or laser. Based on the composition of the media, distinct emission spectrums are given off and captured by the detector 750. Emissions from the media may be directed through the fiber probe 735 to the detector 750. The detector 750 may transmit the captured emission or adsorption spectrum to a user. The user may use software to determine the composition of the media based on the emission or adsorption spectrums. Some examples of analytes that may be measured by the analyte monitor 710 include glucose, lactose, and glutamine.

Fiber probe 735 may include two MMFs used for the input and output of light to and from media in the cell culture chamber 110, 305. The MMFs may have a 90 degree bend near the input/output end. According to embodiments of the present disclosure, one MMF may direct a light beam to the media while the other MMF may capture Raman-scattered light from the media. The distance of the input/output end of the fiber probe 735 from the media may be adjusted for different light beam powers and media illumination areas.

## Claims

1. A remote monitoring system configured to non-invasively measure a cell culture, the system comprising:
a plurality of cell culture layers comprising a cell culture chamber configured to operate as a closed-system, the at least one cell culture chamber having at least one surface to which cells adhere;
at least one monitoring layer comprising an outer wall surrounding a monitoring layer cell culture chamber configured to operate as a closed-system and having at least one surface to which cells adhere, the at least one monitoring layer comprising at least one indentation in the outer wall; and
at least one monitoring module disposed in at least one of the at least one indentation and comprising at least one of a confluence monitor and an analyte monitor.

2. The system of claim 1, further comprising a communication component configured to transmit data from the at least one monitoring module to a remote location.

3. The system of claim 1 or 2, wherein the at least one monitoring module comprises both a confluence monitor and an analyte monitor.

4. The system of any one of claims 1 - 3, wherein at least one of the confluence monitor and the analyte monitor is configured monitor a cell culture chamber of a cell culture layer positioned above or below the at least one monitoring layer.

5. The system of any one of claims 1 - 3, wherein at least one of the confluence monitor and the analyte monitor is configured monitor the monitoring layer cell culture chamber.

6. The system of any one of claims 1-5, wherein the at least one indentation comprises an inner wall configured to contact a front face of a head portion of the at least one monitoring module.

7. The system of any one of claims 1-6, comprising a plurality of monitoring layers.

8. The system of claim 7, comprising between 1 and 50 cell culture layers between two of the plurality of monitoring layers;
comprising between 2 and 40 cell culture layers between two of the plurality of monitoring layers;
comprising between 3 and 35 cell culture layers between two of the plurality of monitoring layers;
comprising between 5 and 30 cell culture layers between two of the plurality of monitoring layers; or
comprising between 10 and 25 cell culture layers between two of the plurality of monitoring layers.

9. The system of any one of claims 1-8, wherein the confluence monitor comprises an optical device configured to capture an image of at least one cell culture chamber;
wherein the confluence monitor comprises at least one lens, at least one mirror and at least one camera;
wherein the confluence monitor comprises a fiber probe, at least one mirror and at least one camera; or
a combination thereof.

10. The system of any one of the preceding claims, wherein the analyte monitor comprises a spectral element configured to emit one or more excitation wavelengths of light and capture emitted light from a media layer within at least one of the cell culture chambers of the plurality of cell culture layers and the monitoring layer cell culture chamber.

11. The system of claim 10, wherein the spectral element is configured to perform Raman spectroscopy on the media layer.

12. The system of claim 10 or 11, wherein the analyte monitor comprises a waveguide and a detector;
wherein the analyte monitor comprises a diffraction grating and lens;
wherein the analyte monitor comprises a fiber probe and a detector; or
a combination thereof.

13. The system of any one of the preceding claims, wherein the analyte monitor is configured to monitor at least one of glucose, lactose, and glutamine within at least one of the cell culture chambers of the plurality of cell culture layers and the monitoring layer cell culture chamber.

14. The system of any one of the preceding claims, wherein the at least one monitoring layer comprises a retaining feature configured to cooperate with the at least one monitoring module to maintain the at least one monitoring module in the at least one indentation.

15. The system of claim 14, wherein the retaining feature comprises at least one clip configured to cooperate with a corresponding at least one receptor of the monitoring module;
wherein the retaining feature comprises a raised channel configured to cooperate with a corresponding notch of the monitoring module; or
a combination thereof.

## Patentansprüche

1. Fernüberwachungssystem, das konfiguriert ist, eine Zellkultur nicht-invasiv zu messen, das System umfassend:
eine Vielzahl von Zellkulturschichten, eine Zellkulturkammer umfassend, die konfiguriert ist, als geschlossenes System zu funktionieren, wobei die zumindest eine Zellkulturkammer zumindest eine Oberfläche aufweist, an der Zellen anhaften;
zumindest eine Überwachungsschicht, eine Außenwand umfassend, die eine Überwachungsschicht-Zellkulturkammer umgibt, die konfiguriert ist, als geschlossenes System zu funktionieren, und die zumindest eine Oberfläche aufweist, an der Zellen anhaften, wobei die zumindest eine Überwachungsschicht zumindest eine Vertiefung in der Außenwand umfasst; und
zumindest ein Überwachungsmodul, das in zumindest einer der zumindest einen Vertiefung angeordnet ist und zumindest eines von einem Konfluenzüberwachungsgerät und einem Analytüberwachungsgerät umfasst.

2. System nach Anspruch 1, ferner eine Kommunikationskomponente umfassend, die konfiguriert ist, Daten von dem zumindest einen Überwachungsmodul an einen entfernten Standort zu übertragen.

3. System nach Anspruch 1 oder 2, wobei das zumindest eine Überwachungsmodul sowohl ein Konfluenzüberwachungsgerät als auch ein Analytüberwachungsgerät umfasst.

4. System nach einem der Ansprüche 1 bis 3, wobei zumindest eines von dem Konfluenzüberwachungsgerät und dem Analytüberwachungsgerät konfiguriert ist, eine Zellkulturkammer einer Zellkulturschicht zu überwachen, die über oder unter der zumindest einen Überwachungsschicht positioniert ist.

5. System nach einem der Ansprüche 1 bis 3, wobei zumindest eines von dem Konfluenzüberwachungsgerät und dem Analytüberwachungsgerät konfiguriert ist, die Überwachungsschicht-Zellkulturkammer zu überwachen.

6. System nach einem der Ansprüche 1 bis 5, wobei die zumindest eine Vertiefung eine Innenwand umfasst, die konfiguriert ist, eine Vorderseite eines Kopfabschnitts des zumindest einen Überwachungsmoduls zu berühren.

7. System nach einem der Ansprüche 1 bis 6, eine Vielzahl von Überwachungsschichten umfassend.

8. System nach Anspruch 7, zwischen 1 und 50 Zellkulturschichten zwischen zwei der Vielzahl von Überwachungsschichten umfassend;
zwischen 2 und 40 Zellkulturschichten zwischen zwei der Vielzahl von Überwachungsschichten umfassend;
zwischen 3 und 35 Zellkulturschichten zwischen zwei der Vielzahl von Überwachungsschichten umfassend;
zwischen 5 und 30 Zellkulturschichten zwischen zwei der Vielzahl von Überwachungsschichten umfassend; oder
zwischen 10 und 25 Zellkulturschichten zwischen zwei der Vielzahl von Überwachungsschichten umfassend.

9. System nach einem der Ansprüche 1 bis 8, wobei das Konfluenzüberwachungsgerät eine optische Vorrichtung umfasst, die konfiguriert ist, ein Bild von zumindest einer Zellkulturkammer aufzunehmen;
wobei das Konfluenzüberwachungsgerät zumindest eine Linse, zumindest einen Spiegel und zumindest eine Kamera umfasst;
wobei das Konfluenzüberwachungsgerät eine faseroptische Sonde, zumindest einen Spiegel und zumindest eine Kamera umfasst; oder
eine Kombination davon.

10. System nach einem der vorhergehenden Ansprüche, wobei das Analytüberwachungsgerät ein Spektralelement umfasst, das konfiguriert ist, eine oder mehrere Anregungswellenlängen von Licht zu emittieren und emittiertes Licht von einer Medienschicht innerhalb zumindest einer der Zellkulturkammern der Vielzahl von Zellkulturschichten und der Überwachungsschicht-Zellkulturkammer aufzunehmen.

11. System nach Anspruch 10, wobei das Spektralelement konfiguriert ist, Raman-Spektroskopie an der Medienschicht durchzuführen.

12. System nach Anspruch 10 oder 11, wobei das Analytüberwachungsgerät einen Wellenleiter und einen Detektor umfasst
wobei das Analytüberwachungsgerät ein Beugungsgitter und eine Linse umfasst;
wobei das Analytüberwachungsgerät eine faseroptische Sonde und einen Detektor umfasst; oder eine Kombination davon.

13. System nach einem der vorhergehenden Ansprüche, wobei das Analytüberwachungsgerät konfiguriert ist, zumindest eines von Glukose, Laktose und Glutamin innerhalb zumindest einer der Zellkulturkammern der Vielzahl von Zellkulturschichten und der Überwachungsschicht-Zellkulturkammer zu überwachen.

14. System nach einem der vorhergehenden Ansprüche, wobei die zumindest eine Überwachungsschicht ein Rückhaltemerkmal umfasst, das konfiguriert ist, mit dem zumindest einen Überwachungsmodul zusammenzuwirken, das zumindest eine Überwachungsmodul in der zumindest einen Vertiefung zu halten.

15. System nach Anspruch 14, wobei das Rückhaltemerkmal zumindest eine Klammer umfasst, die dazu konfiguriert ist, mit einem entsprechenden zumindest einer Aufnahme des Überwachungsmoduls zusammenzuwirken;
wobei das Rückhaltemerkmal einen erhöhten Kanal umfasst, der konfiguriert ist, mit einer entsprechenden Kerbe des Überwachungsmoduls zusammenzuwirken; oder
eine Kombination davon.

## Revendications

1. Système de surveillance à distance conçu pour mesurer de manière non invasive une culture cellulaire, le système comprenant :
une pluralité de couches de culture cellulaire comprenant une chambre de culture cellulaire conçue pour fonctionner en tant que système fermé, l'au moins une chambre de culture cellulaire possédant au moins une surface à laquelle les cellules adhèrent ;
au moins une couche de surveillance comprenant une paroi externe entourant une chambre de culture cellulaire de couche de surveillance conçue pour fonctionner en tant que système fermé et possédant au moins une surface à laquelle les cellules adhèrent, l'au moins une couche de surveillance comprenant au moins une indentation dans la paroi externe ; et
au moins un module de surveillance disposé dans au moins l'une des au moins une indentation et comprenant au moins l'un d'un dispositif de surveillance de confluence et d'un dispositif de surveillance de substance à analyser.

2. Système selon la revendication 1, comprenant en outre un composant de communication conçu pour transmettre des données de l'au moins un module de surveillance à un emplacement distant.

3. Système selon la revendication 1 ou 2, ledit au moins un module de surveillance comprenant à la fois un dispositif de surveillance de confluence et un dispositif de surveillance de substance à analyser.

4. Système selon l'une quelconque des revendications 1 à 3, au moins l'un du dispositif de surveillance de confluence et du dispositif de surveillance de substance à analyser étant conçu pour surveiller une chambre de culture cellulaire d'une couche de culture cellulaire positionnée au-dessus ou au-dessous de l'au moins une couche de surveillance.

5. Système selon l'une quelconque des revendications 1 à 3, au moins l'un du dispositif de surveillance de confluence et du dispositif de surveillance de substance à analyser étant conçu pour surveiller la chambre de culture cellulaire de couche de surveillance.

6. Système selon l'une quelconque des revendications 1 à 5, ladite au moins une indentation comprenant une paroi interne conçue pour entrer en contact avec une face avant d'une partie tête de l'au moins un module de surveillance.

7. Système selon l'une quelconque des revendications 1 à 6, comprenant une pluralité de couches de surveillance.

8. Système selon la revendication 7, comprenant entre 1 et 50 couches de culture cellulaire entre deux de la pluralité de couches de surveillance ;
comprenant entre 2 et 40 couches de culture cellulaire entre deux de la pluralité de couches de surveillance ;
comprenant entre 3 et 35 couches de culture cellulaire entre deux de la pluralité de couches de surveillance ;
comprenant entre 5 et 30 couches de culture cellulaire entre deux de la pluralité de couches de surveillance ; ou
comprenant entre 10 et 25 couches de culture cellulaire entre deux de la pluralité de couches de surveillance.

9. Système selon l'une quelconque des revendications 1 à 8, ledit dispositif de surveillance de confluence comprenant un dispositif optique conçu pour capturer une image d'au moins une chambre de culture cellulaire ;
ledit dispositif de surveillance de confluence comprenant au moins une lentille, au moins un miroir et au moins une caméra ;
ledit dispositif de surveillance de confluence comprenant une sonde à fibre, au moins un miroir et au moins une caméra ; ou
une combinaison de ceux-ci.

10. Système selon l'une quelconque des revendications précédentes, ledit dispositif de surveillance de substance à analyser comprenant un élément spectral conçu pour émettre une ou plusieurs longueurs d'onde d'excitation de lumière et capturer la lumière émise à partir d'une couche de support à l'intérieur d'au moins l'une des chambres de culture cellulaire de la pluralité de couches de culture cellulaire et de la chambre de culture cellulaire de couche de surveillance.

11. Système selon la revendication 10, ledit élément spectral étant conçu pour réaliser une spectroscopie Raman sur la couche de support.

12. Système selon la revendication 10 ou 11, ledit dispositif de surveillance de substance à analyser comprenant un guide d'ondes et un détecteur ;
ledit dispositif de surveillance de substance à analyser comprenant un réseau de diffraction et une lentille ;
ledit dispositif de surveillance de substance à analyser comprenant une sonde à fibre et un détecteur ; ou
une combinaison de ceux-ci.

13. Système selon l'une quelconque des revendications précédentes, ledit dispositif de surveillance de substance à analyser étant conçu pour surveiller au moins l'un du glucose, du lactose et de la glutamine dans au moins l'une des chambres de culture cellulaire de la pluralité de couches de culture cellulaire et de la chambre de culture cellulaire de couche de surveillance.

14. Système selon l'une quelconque des revendications précédentes, ladite au moins une couche de surveillance comprenant un élément de retenue conçu pour coopérer avec l'au moins un module de surveillance pour maintenir l'au moins un module de surveillance dans l'au moins une indentation.

15. Système selon la revendication 14, ledit élément de retenue comprenant au moins un clip conçu pour coopérer avec au moins un récepteur correspondant du module de surveillance ;
ledit élément de retenue comprenant un canal surélevé conçu pour coopérer avec une encoche correspondante du module de surveillance ; ou
une combinaison de ceux-ci.
